Europäisches Patentamt

European Patent Office

Office européen des brevets

(11) Publication number: **0 373 507**
**A1**

## EUROPEAN PATENT APPLICATION

(21) Application number: **89122572.4**

(22) Date of filing: **07.12.89**

(51) Int. Cl.5: **A61K 31/455, A61K 31/66,**
**A61K 31/365, A61K 31/415,**
**A61K 31/19, A61K 31/40,**
**A61K 31/38, A61K 31/34**

(30) Priority: **12.12.88 US 282647**

(43) Date of publication of application:
**20.06.90 Bulletin 90/25**

(84) Designated Contracting States:
**AT BE CH DE ES FR GB GR IT LI LU NL SE**

(71) Applicant: **E.R. SQUIBB & SONS, INC.**
**P.O.Box 4000**
**Princeton New Jersey 08543-4000(US)**

(72) Inventor: **Dennick, Leonard G.**
**475 Riverside Drive**
**Princeton New Jersey 08540(US)**

(74) Representative: **Josif, Albert et al**
**Baaderstrasse 3**
**D-8000 München 5(DE)**

(54) Combination of an HMG CoA reductase inhibitor and other type of serum cholesterol reducing agent and method for lowering serum cholesterol using such combination.

(57) A pharmaceutical combination is provided which includes an inhibitor of the enzyme 3-hydroxy-3-methyl-glutaryl coenzyme A (HMG CoA) reductase, such as lovastatin, pravastatin or velostatin, and a pharmaceutical which reduces serum cholesterol and/or inhibits cholesterol biosynthesis by a mechanism other than inhibiting production of the enzyme HMG CoA reductase, namely, probucol or niacin. A method for reducing serum cholesterol or inhibiting formation of or treating atherosclerosis using the above combination is also provided.

EP 0 373 507 A1

## COMBINATION OF AN HMG CoA REDUCTASE INHIBITOR ANO OTHER TYPE OF SERUM CHOLESTEROL REDUCING AGENT AND METHOD FOR LOWERING SERUM CHOLESTEROL USING SUCH COMBINATION

The present invention relates to a combination of an inhibitor of 3-hydroxy-3-methylglutaryl coenzyme A (HMG CoA) reductase and a pharmaceutical which reduces serum cholesterol other than by inhibiting the enzyme HMG CoA reductase, and to a method for lowering serum cholesterol and/or preventing or treating atherosclerosis by administering such combination.

There are several different classes of compounds which have serum cholesterol lowering properties. Some of these compounds are inhibitors of the enzyme HMG CoA reductase which is essential in the production of cholesterol, such as mevastatin (disclosed in U. S. Patent No. 3,983,140), lovastatin also referred to as mevinolin (disclosed in U. S. Patent No. 4,231,938), pravastatin (disclosed in U. S. Patent No. 4,346,227) and velostatin also referred to as synvinolin (disclosed in U. S. Patents Nos. 4,448,784 and 4,450,171).

Other compounds which lower serum cholesterol may do so by an entirely different mechanism than the HMG CoA reductase inhibitors. For example, serum cholesterol may be lowered through the use of bile acid seguestrants such as cholestyramine, colestipol, DEAE-Sephadex and poly(diallylmethylamine) derivatives (such as disclosed in U. S. Patents Nos. 4,759,923 and 4,027,009) or through the use of antihyperlipoproteinemics such as probucol and gemfibrozil which apparently lower serum lower density lipoproteins (LDL) and/or converts LDL into high density lipoproteins (HDL).

U. S. patent No. 4,759,923 mentioned above discloses that poly(diallylmethylamine) derivatives which are bile salt seguestrants may be used in conjunction with drugs which reduce serum cholesterol by mechanisms other than sequestration, such as clofibrate, nicotinic acid, probucol, neomycin, p-aminosalicylic acid or mevinolin (also referred to as lovastatin).

In accordance with the present invention, a pharmaceutical combination is provided for use in reducing serum cholesterol and in inhibiting formation of or treating atherosclerosis, which combination is formed of an inhibitor of the enzyme 3-hydroxy-3-methylglutaryl coenzyme A (HMG CoA) reductase and a pharmaceutical (also referred to as other serum cholesterol lowering agent) which reduces serum cholesterol and/or inhibits cholesterol biosynthesis by a mechanism other than by inhibiting production of the enzyme HMG CoA reductase, such as an antihyperlipoproteinemic agent namely probucol or niacin which inhibits formation of LDL or converts LDL to HDL. The HMG CoA reductase inhibitor will be employed in a weight ratio to the "pharmaceutical" of within the range of from about 0.001:1 to about 1000:1 and preferably from about 0.05:1 to about 100:1.

In addition, in accordance with the present invention, a method is provided for lowering serum cholesterol or inhibiting formation of or treating atherosclerosis wherein a therapeutically effective amount of the above combination is systemically, such as orally or parenterally, administered over a prolonged period.

It has been found that the combination of the HMG CoA reductase inhibitor and other serum cholesterol lowering agent, which works by a mechanism other than inhibiting HMG CoA reductase, is a surprising and unique concept in inhibiting or treating elevated cholesterol and/or atherosclerosis in that it may provide additional anti-cholesterolemic effects over that which may be obtained using each of the components of the combination alone. In addition, the combination of the invention which includes compounds with different mechanisms of action, may be used to effectively treat cholesterol-related diseases of multiple etiology.

The HMG CoA reductase inhibitors suitable for use herein include, but are not limited to, mevastatin and related compounds as disclosed in U. S. Patent No. 3,983,140, lovastatin (mevinolin) and related compounds as disclosed in U. S. Patent No. 4,231,938, pravastatin and related compounds such as disclosed in U. S. Patent No. 4,346,227, velostatin (synvinolin) and related compounds as disclosed in U. S. Patents Nos. 4,448,784 and 4,450,171, with lovastatin, pravastatin or velostatin being preferred. Other HMG CoA reductase inhibitors which may be employed herein include, but are not limited to, eptastatin, fluindostatin (Sandoz XU-62-320), pyrazole analogs of mevalonolactone derivatives as disclosed in U. S. Patent No. 4,613,610, indene analogs of mevalonolactone derivatives as disclosed in PCT application WO 86/03488, 6-[2-(substituted-pyrrol-1-yl)alkyl]pyran-2-ones and derivatives thereof as disclosed in U. S. Patent No. 4,647,576, Searle's SC-45355 (a 3-substituted pentanedioic acid derivative) dichloroacetate, imidazole analogs of mevalonolactone as disclosed in PCT application WO 86/07054, 3-carboxy-2-hydroxy-propane-phosphonic acid derivatives as disclosed in French Patent No. 2,596,393, 2,3-di-substituted pyrrole, furan and thiophene derivatives as disclosed in European Patent Application No. 0221025, naphthyl analogs of mevalonolactone as disclosed in U. S. Patent No. 4,686,237, octahydronaphthalenes such as disclosed in U. S. Patent No. 4,499,289, keto analogs of mevinolin (lovastatin) as disclosed in European Patent Application No. 0,142,146 A2, as well as other known HMG CoA reductase inhibitors.

The "pharmaceutical" or other serum cholesterol lowering agents which function other than by inhibiting the enzyme HMG CoA reductase suitable for use herein include, but are not limited to, antihyperlipoproteinemic agents such as probucol or niacin.

Preferred are combinations of lovastatin, pravastatin or velostatin with probucol or niacin.

The disclosure of the above-mentioned patents and patent applications are incorporated herein by reference.

In carrying out the method of the present invention, the combination of the invention may be administered to mammalian species, such as monkeys, dogs, cats, rats, humans, etc. and as such may be incorporated in a conventional systemic dosage form, such as a tablet, capsule, elixir or injectable. The above dosage forms will also include the necessary carrier material, excipient, lubricant, buffer, antibacterial, bulking agent (such as mannitol), anti-oxidants (ascorbic acid of sodium bisulfite) or the like. Oral dosage forms are preferred, although parenteral forms are quite satisfactory as well.

The dose administered must be carefully adjusted according to age, weight and condition of the patient, as well as the route of administration, dosage form and regimen and the desired result.

Thus, for oral administration, a satisfactory result may be obtained employing the HMG CoA reductase inhibitor in dosages employed, for example, for lovastatin as indicated in the Physician's Desk Reference, such as in an amount within the range of from about 1 to 2000 mg and preferably from about 4 to about 200 mg in combination with the other serum cholesterol lowering agent in dosages normally employed as indicated in the Physician's Desk Reference, for each of such agents such as in an amount within the range of from about 2 mg to about 7500 mg and preferably from about 2 mg to about 4000 mg with the HMG CoA reductase inhibitor and other serum cholesterol lowering agent being employed together in the same oral dosage form or in separate oral dosage forms taken at the same time.

A preferred oral dosage form, such as tablets or capsules, will contain the HMG CoA reductase inhibitor in an amount of from about 0.1 to about 100 mg, preferably from about 5 to about 80 mg, and more preferably from about 10 to about 40 mg, and the other serum cholesterol lowering agent in an amount of from about 2 to about 3000 mg, preferably from about 2 to about 2000 mg.

The composition described above may be administered in the dosage forms as described above in single or divided doses of one to four times daily. It may be advisable to start a patient on a low dose combination and work up gradually to a high dose combination.

Tablets of various sizes can be prepared, e.g., of about 2 to 2000 mg in total weight, containing one or both of the active substances in the ranges described above, with the remainder being a physiologically acceptable carrier of other materials according to accepted pharmaceutical practice. These tablets can, of course, be scored to provide for fractional doses. Gelatin capsules can be similarly formulated.

Liquid formulations can also be prepared by dissolving or suspending one or the combination of active substances in a conventional liquid vehicle acceptable for pharmaceutical administration so as to provide the desired dosage in one to four teaspoonsful.

Such dosage forms can be administered to the patient on a regimen of one to four doses per day.

According to another modification, in order to more finely regulate the dosage schedule, the active substances may be administered separately in individual dosage units at the same time or carefully coordinated times. Since blood levels are built up and maintained by a regulated schedule of administration, the same result is achieved by the simultaneous presence of the two substances. The respective substances can be individually formulated in separate unit dosage forms in a manner similar to that described above.

Fixed combinations of HMG CoA reductase inhibitor and other serum cholesterol lowering agent are more convenient and are preferred, especially in tablet or capsule form for oral administration.

In formulating the compositions, the active substances, in the amounts described above, are compounded according to accepted pharmaceutical practice with a physiologically acceptable vehicle, carrier, excipient, binder, preservative, stabilizer, flavor, etc., in the particular type of unit dosage form.

Illustrative of the adjuvants which may be incorporated in tablets are the following: a binder such as gum tragacanth, acacia, corn starch or gelatin; an excipient such as dicalcium phosphate or cellulose; a disintegrating agent such as corn starch, potato starch, alginic acid or the like; a lubricant such as stearic acid or magnesium stearate; a sweetening agent such as sucrose, aspartame, lactose or saccharin; a flavoring agent such as orange, peppermint, oil of wintergreen or cherry. When the dosage unit form is a capsule, it may contain in addition to materials of the above type a liquid carrier such as a fatty oil. Various other materials may be present as coatings or to otherwise modify the physical form of the dosage unit. For instance, tablets or capsules may be coated with shellac, sugar or both. A syrup of elixir may contain the active compound, water, alcohol or the like as the carrier, glycerol as solubilizer, sucrose as sweetening agent, methyl and propyl parabens as preservatives, a dye and a flavoring such as cherry or orange.

Some of the active substances described: above form commonly known, pharmaceutically acceptable salts such as alkali metal and other common basic salts or acid addition salts, etc. References to the base substances are therefore intended to include those common salts known to be substantially equivalent to the parent compound.

The formulations as described above will be administered for a prolonged period, that is, for as long as the potential for elevated serum cholesterol and atherosclerosis remains or the symptoms continue. Sustained release forms of such formulations which may provide such amounts biweekly, weekly, monthly and the like may also be employed. A dosing period of at least one to two weeks are required to achieve minimal benefit.

The following Examples represent preferred embodiments of the present invention. All temperatures are expressed in degrees Centigrade unless otherwise indicated and all mesh sizes are U.S. Standard ASTME.

## Example 1

A pravastatin formulation in the form of tablets having the following composition was prepared as described below.

| Ingredient | Parts by Weight |
|---|---|
| Pravastatin | 7 |
| Lactose | 67 |
| Microcrystalline cellulose | 20 |
| Croscarmellose sodium | 2 |
| Magnesium stearate | 1 |
| Magnesium oxide | 3 |

Pravastatin, magnesium oxide and a fraction (30%) of the lactose were mixed together for 2 to 10 minutes employing a suitable mixer. The resulting mixture was passed through a #12 to #40 mesh size screen. Microcrystalline cellulose, croscarmellose sodium and the remaining lactose were added and the mixture was mixed for 2 to 10 minutes. Thereafter, magnesium stearate was added and mixing was continued for 1 to 3 minutes.

The resulting homogeneous mixture was then compressed into tablets each containing 5 mg, 10 mg, 20 or 40 mg pravastatin.

Probucol tablets containing 250 mg probucol are prepared employing conventional procedures containing the following additional ingredients as set out in the 1988 PDR: corn starch, ethyl cellulose, glycerin, hydroxypropyl cellulose, hydroxypropylmethyl cellulose 2910, iron oxide, lactose, magnesium stearate, microcrystalline cellulose, polysorbate 80, talc and titanium dioxide.

The pravastatin tablets and probucol tablets may be administered as a combination in accordance with the teachings of the present invention to lower serum cholesterol and/or treat atherosclerosis. In addition, the pravastatin and probucol tablets may be ground up into powders and used together in a single capsule.

## Examples 2 and 3

Lovastatin tablets are prepared employing conventional pharmaceutical techniques containing 20 mg lovastatin, cellulose, color, lactose, magnesium stearate and starch and butylated hydroxyanisole as a preservative as described in the 1988 PDR.

The lovastatin tablets may be employed in combination with the probucol tablets (described in Example 1) in separate or combined dosage forms to treat elevated serum cholesterol or atherosclerosis in accordance with the present invention.

## Examples 4 to 5

Pravastatin tablets, or lovastatin tablets described in Examples 1 and 2, respectively, or velostatin tablets may be employed in combination with niacin capsules containing 500 mg niacin and inactive ingredients as described in the 1988 PDR. The pravastatin or lovastatin and niacin may be employed in separate dosage forms or combined in a single capsule form to lower elevated serum cholesterol or treat atherosclerosis in accordance with the present invention.

**Claims**

1. A pharmaceutical combination comprising an inhibitor of the enzyme 3-hydroxy-3-methylglutaryl coenzyme A (HMG CoA) reductase and probucol or niacin.

2. The combination as defined in Claim 1 wherein said inhibitor of the enzyme HMG CoA reductase is mevastatin, lovastatin, pravastatin, velostatin or eptastatin.

3. The combination as defined in Claim 1 wherein said inhibitor of the enzyme HMG CoA reductase is a pyrazole analog of a mevalonolactone, an indene analog of mevalonolactone, a 3-carboxy-2-hydroxy-propane-phosphonic acid derivative, a 6-[2-(substituted-pyrrol-1-yl)alkyl]pyran-2-one, an imidazole analog of mevalonolactone or a heterocyclic analog of mevalonolactone, a naphthyl analog of mevalonolactone, an octahydro-naphthalene, fluindostatin, a keto analog of lovastatin or a 2,3-di-substituted pyrrole, furan or thiophene.

4. The combination as defined in CLaim 1 wherein the inhibitor of the enzyme HMG CoA reductase is present in a weight ratio to probucol or niacin of within the range of from about 0.001:1 to about 1000:1.

5. The combination as defined in Claim 1 wherein the inhibitor of the enzyme HMG CoA reductase is lovastatin, pravastatin or velostatin in combination with probucol.

6. A combination comprising an inhibitor of the enzyme 3-hydroxy-3-methyl-glutaryl coenzyme A (HMG CoA) reductase and probucol or niacin for use in lowering serum cholesterol or inhibiting formation of or treating atherosclerosis in a patient in need of such treatment.

7. A combination as defined in claim 6 wherein said inhibitor of enzyme HMG CoA reductase is mevastatin, lovastatin, pravastatin, velostatin or eptastatin.

8. A combination as defined in claim 6 wherein the inhibitor of the enzyme HMG CoA reductase is lovastatin, pravastatin or velostatin in combination with probucol.

9. A hypocholesterolemic or hypolipemic composition comprising a combination as defined in claim 1 and a pharmaceutically acceptable carrier therefor.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DIALOG 6745167 MEDLINE 89047167; J.A. TOBERT: "Efficacy and long-term adverse effect pattern of lovastatin", & AM. J. CARDIOL. November 1988, vol. 62, (15), pages 28J-34J<br>* Whole abstract *<br>--- | 1,2 | A 61 K 31/455<br>A 61 K 31/66<br>A 61 K 31/365<br>A 61 K 31/415<br>A 61 K 31/19<br>A 61 K 31/40<br>A 61 K 31/38<br>A 61 K 31/34 |
| X | DIALOG 6648621 MEDLINE 88293621; A. YAMAMOTO et al.: "Escape phenomenon occurs by lowering cholesterol with a hydroxymethylglutaryl coenzyme A (HMG-CoA) reductase inhibitor in patients with familial hypercholesterolemia", & ATHEROSCLEROSIS, June 1988, vol. 71, nos. 2-3, pages 257-260<br>* Whole abstract *<br>--- | 1,3,5 | |
| P,X | DIALOG 6802816 MEDLINE 89104816; D.R. ILLINGWORTH et al.: "Treatment of heterozygous familial hypercholesterolemia with lipid-lowering drugs", & ARTERIOSCLEROSIS (UNITED STATES), January-February 1989, vol. 9, (suppl. 1) pages I121-34<br>* Whole abstract *<br>--- | 1,2 | **TECHNICAL FIELDS SEARCHED (Int. Cl.5)**<br><br>A 61 K |
| A | EUROPEAN HEART JOURNAL, vol. 8, 1987, pages 103-111, London, GB; D.R. ILLINGWORTH: "Long term administration of lovastatin in the treatment of hypercholesterolaemia"<br>* Abstract *<br>----- | 1,2 | |

The present search report has been drawn up for all claims

| Place of search | Date of completion of the search | Examiner |
|---|---|---|
| THE HAGUE | 27-02-1990 | BERTOCCHI C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................
& : member of the same patent family, corresponding document

EPO FORM 1503 03.82 (P0401)